# EUROPEAN PATENT SPECIFICATION

(11) **EP 4 404 877 B1**
(45) Date of publication and mention of the grant of the patent: **18.06.2025**
(21) Application number: 22783028.8
(22) Date of filing: 16.09.2022
(51) Int. Cl.: A61F 2/64, A61F 2/72, A61F 2/70, A61F 2/60

(54) **ACTIVE PROSTHETIC JOINT**
AKTIVES PROTHESENGELENK
ARTICULATION PROTHÉTIQUE ACTIVE

(30) Priority: 23.09.2021 IT 202100024512
(43) Date of publication of application: 31.07.2024
(73) Proprietor: Fondazione Istituto Italiano di Tecnologia, 16163 Genova (IT); I.N.A.I.L. ISTITUTO NAZIONALE PER L'ASSICURAZIONE CONTRO GLI INFORTUNI SUL LAVORO, 00187 Roma (IT)
(72) Inventor: GUERCINI, Lorenzo, Genova (IT); MILANDRI, Giovanni Sergio, Londra (GB); TRAVERSO, Simone, Genova (IT); LAFFRANCHI, Matteo, 16163 Genova (IT); DE MICHIELI, Lorenzo, 16163 Genova (IT)
(74) Representative: Lunati & Mazzoni S.r.L.
(86) International application number: PCT/IB2022/058755
(87) International publication number: WO 2023/047256

(56) References cited:
- AU-A1- 2010 208 020
- US-A1- 2013 261 766
- US-A1- 2019 175 365

## Description

The present invention relates to an active prosthetic joint of the type specified in the preamble to the first claim.

In particular, the invention introduces a device, preferably prosthetic, configured to control a rotation of a robotic limb (in particular a portion of a limb such as a leg) relative to a second element such as a torso or a different portion of a limb such as a thigh. Preferably the joint is a prosthetic knee.

Currently in the field of robotic prosthetics, one of the biggest problems is balancing product design between performance (in terms of usable torque, maximum actuator speed and energy consumption) and the overall dimensions and weight of the product.

An obvious example of such a problem lies in prostheses simulating a preferably propulsive joint where there are considerable difficulties in fulfilling the performance diversity requirements in the various phases of movement.

This is convenient in a propulsive knee joint where pitch and bulk requirements clash with functional ones. In fact, the human quadriceps, which actuates the knee joint, on a fast walk averages a peak power of 1.7 W/kg (130 watts for an 80 kg patient) while on an incline this number increases significantly to reach an average peak power of 2.4 W/kg (192 watts for an 80 kg patient).

In an attempt to address this, the products currently on the market (e.g. Power Knee, Ossur) have unbalanced performance towards propulsive torque, to the detriment of maximum joint speed.

The known technique described includes some major drawbacks.

In particular, the well-known active prosthetic joints are bulky and heavy, so an operator usually prefers passive (i.e. non-motorised) prosthetic joints that are light and unobtrusive.

This aspect, and thus the preference for passive prosthetic joints, is accentuated by the considerable difficulties of control and management encountered by the operator.

A not insignificant drawback is that the well-known active prosthetic joints have particularly complex mechanics and therefore have high costs in both production and maintenance.

In this situation, the technical task at the basis of the present invention is to devise an active prosthetic joint that can substantially obviate at least part of the aforementioned drawbacks. US 2013/261766 shows a prosthetic joint configured to mutually rotate a first prosthesis and a second prosthesis according to the preamble of claim 1.

Within this technical task, it is an important aim of the invention to obtain a lightweight and space-saving active prosthetic joint

Another important aim of the invention is to realise an active prosthetic joint characterised by ease of use and featuring relatively simple mechanics and low production and maintenance costs.

The specified technical task and purposes are achieved by a prosthetic joint as claimed in the attached claim 1. Examples of preferred embodiments are described in the dependent claims.

The features and advantages of the invention are clarified below by a detailed description of preferred embodiments of the invention, with reference to the accompanying drawings, in which:

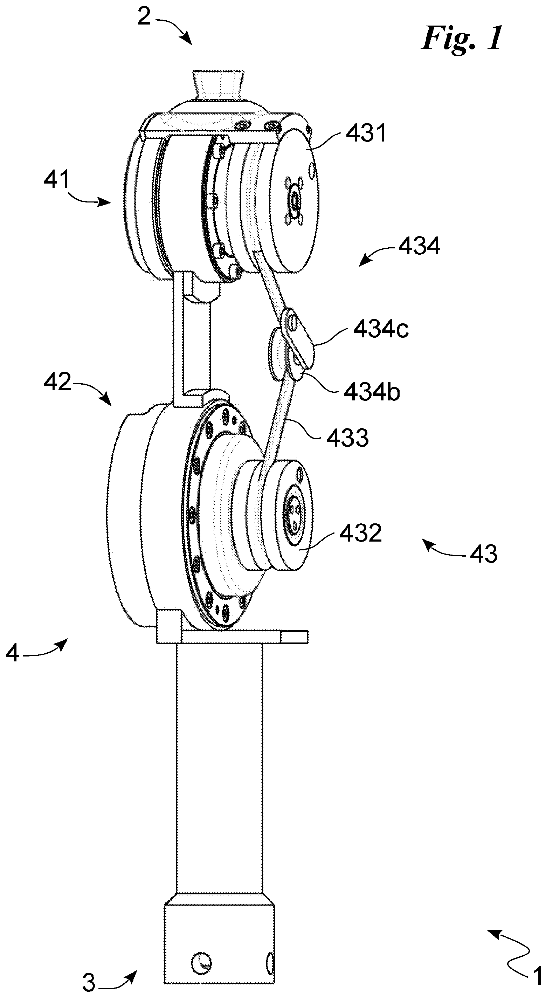

the Fig. 1 shows, to scale, a prosthetic joint according to the invention;

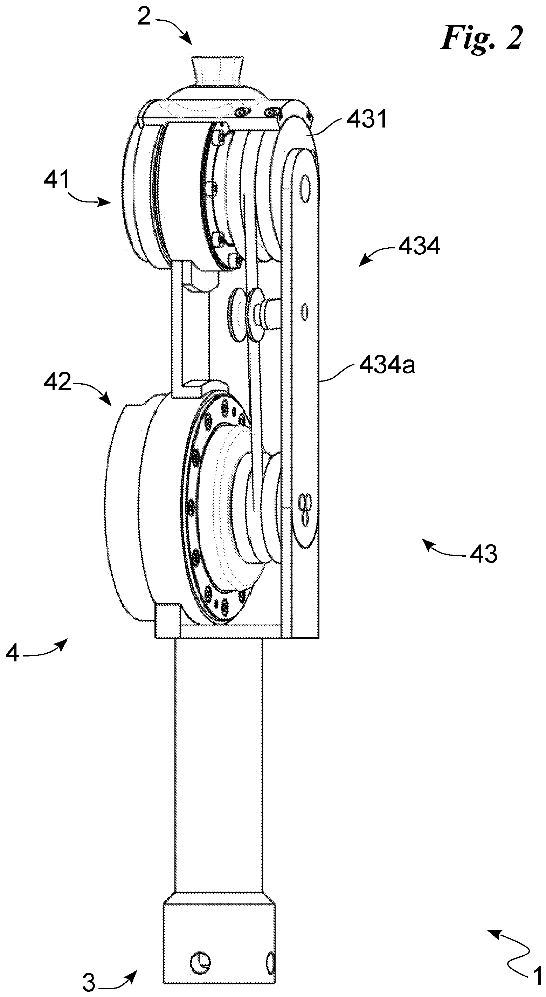

the Fig. 2 illustrates, to scale, a prosthetic joint according to the invention in a second configuration;

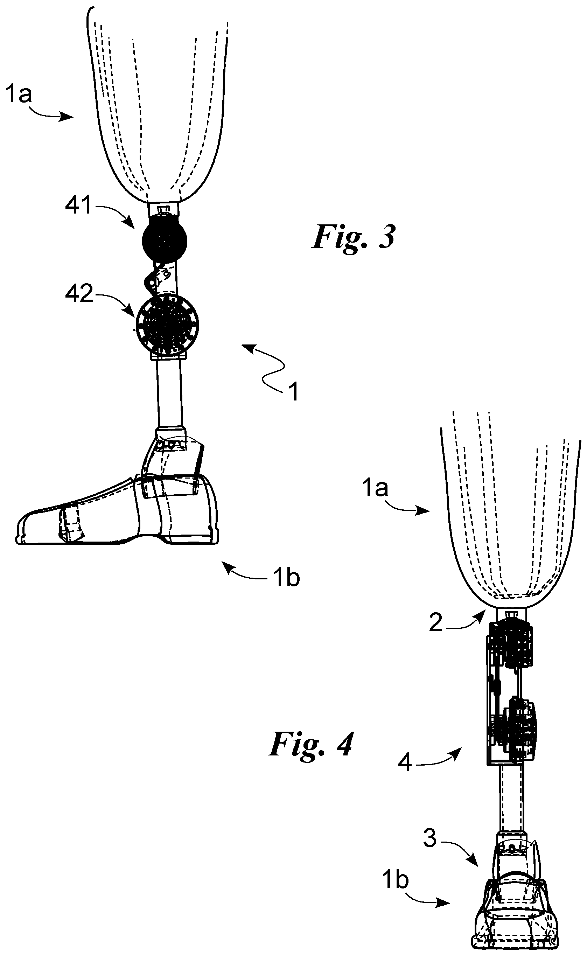

the **Fig. 3** shows, to scale, a side view of a prosthetic joint in use; and
the **Fig. 4** shows, to scale, a frontal view of Fig. 3.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

Unless otherwise indicated, "perpendicular", "transverse", "parallel" or "normal" or other terms of geometric positioning between geometric elements (e.g. axes, directions and straight lines) are to be understood with reference to their reciprocal geometric position between the corresponding projections. These projections are defined on a single plane parallel to the plane(s) of location of said geometric elements.

The measurements and data reported in this text are to be considered, unless otherwise indicated, as performed in the International Standard Atmosphere ICAO (ISO 2533:1975).

Unless otherwise indicated, "perpendicular", "transverse", "parallel" or "normal" or other terms of geometric positioning between geometric elements (e.g. axes, directions and straight lines) are to be understood with reference to their reciprocal geometric position between the corresponding projections. These projections are defined on a single plane parallel to the plane(s) of location of said geometric elements.

With reference to the Figures, the prosthetic joint according to the invention is indicated globally by the number **1.**

It is configured to be connected to a first prosthesis **1a** and a second prosthesis 1b and in detail interposed between said prostheses 1a and 1b.

The first prosthesis 1a may be identified in a torso or a limb (particularly a portion of a limb such as a thigh). In some, the first prosthesis 1a may be a prosthetic socket. The second prosthesis 1b can be identified in a robotic limb and in particular in a portion of a limb such as a leg and/or a foot.

It is configured to command a rotation between a first prosthesis 1a and a second prosthesis 1b. In detail, it is configured to command a rotation of the first prosthesis 1a with respect to the second prosthesis 1b and appropriately with respect to the same joint 1

Preferably the joint 1 is a knee that allows rotation with respect to the knee prosthesis and the femoral stump.

The prosthetic joint 1 can define an angle of rotation, i.e. a maximum amplitude of rotation that can be performed by the same joint 1. This angle can be almost less than 360° in detail to 180° and appropriately between 90° and 135°.

The prosthetic joint 1 may comprise an attachment **2** of the joint 1 to the first prosthesis 1a and appropriately an additional attachment **3** of the joint 1 to the second prosthesis 1b. The attachment 2 is between joint 1 and first prosthesis 1a and the additional attachment 3 between joint 1 and second prosthesis 1b.

For example, the attachment 3 may be a female pyramid or other attachment configured to allow connection with a rigid prosthetic foot.

The prosthetic joint 1 may include a movement member **4** configured to control a rotation between attachments 2 and 3 and thus between prostheses 1a and 1b. More precisely, the movement member 4 is configured to control a rotation of the first joint 2 and 3 and thus of the first prosthesis 1a with respect to the second prosthesis 1b and appropriately with respect to the same joint 1

The transmission member 4 can define a thrust torque for said rotation. For example, in the case of a prosthetic joint 1 identifiable in a knee, the thrust torque defines the power that the joint 1 expresses by allowing, for example, a walk or a climb.

The member 4 can define a rotation speed for said rotation. For example, in the case of the joint 1 identifiable in a knee, this speed represents the speed that prosthetic joint 1 expresses during, for example, a walk or a climb.

The transmission member 4 may comprise a first actuator **41** defining for this rotation a first torque and appropriately a first speed.

The first torque identifies the maximum torque that can be expressed by the first actuator 41. The first torque can be substantially less than 75 Nm, in detail roughly between 75 Nm and 25 Nm. More specifically, it is substantially equal to 50 Nm. The first speed identifies the maximum rotation speed when the first actuator 41 expresses said first torque.

It can be substantially at least equal to 30 rpm, in detail roughly between 100 rpm and 50 rpm. More specifically, the first speed is essentially equal to 80 rpm.

The first actuator 41 may comprise a first preferably electric motor and appropriately a first preferably harmonic gearbox.

The first motor can be electric.

The transmission member 4 may include a second actuator **42** defining for this rotation a second torque and appropriately a second speed.

The second torque identifies the maximum torque that can be expressed by the second actuator 42.

The second pair may be substantially larger than the first pair.

It can be substantially at least equal to 50 Nm, in detail roughly between 50 Nm and 150 Nm. More specifically, the second torque can be substantially equal to 100 Nm. The second speed identifies the maximum rotation speed when the second actuator 42 expresses said second torque.

The second speed may be substantially lower than the first speed.

It can be substantially less than 50 rpm, in detail roughly between 15 rpm and 50 rpm. More specifically, the second speed is essentially 20 rpm.

To be more precise, it should be noted that preferably the torque and speed numbers mentioned above are peak values that the actuators can generate and therefore not a fixed torque.

The second actuator 42 may comprise a second preferably electric motor and appropriately a second preferably harmonic gear.

The second motor can be electric.

Transmission gear 4 may include a kinematic mechanism **43** configured to utilise actuators 41 and 42 to drive said rotation.

The kinematic mechanism 43 is configured to selectively place actuators 41 and 42 in kinematic connection (i.e., a connection such that the motion exiting at least one actuator 41 and/or 42 drives said rotation) with the first attachment 2.

Preferably it is configured to define a low-thrust configuration and a high-thrust configuration.

In this configuration, the first actuator 41 is kinematically connected to the attachment 2 and the second actuator 42 is kinematically disconnected so that only the first actuator 41 controls this rotation.

Therefore, in this configuration, the first actuator 41 can rotate appropriately by an angle almost no greater than said angle of rotation. The second 42 remains substantially stationary.

In the low-thrust configuration, the maximum rotational torque that can be expressed by drive 4 can be substantially no higher than the first torque.

Preferably in the low-thrust configuration, the maximum rotational speed that can be expressed by the transmission member 4 is substantially no higher than the first speed.

In the high-thrust configuration, the kinematic mechanism 43 places both actuators 41 and 42 in kinematic connection with port 2 so that both the first actuator 41 and the second actuator 42 control this rotation.

In this configuration, the actuators 41 and 42 can rotate simultaneously and in detail with the same speed for at least said angle of rotation. Preferably in the high thrust configuration the actuators 41 and 42 rotate in one direction simultaneously and suitably with the same rotation speed for at least said angle of rotation. In the other direction the actuators 41 and 42 can rotate independently of each other for at least said angle of rotation.

In the high-thrust configuration, the maximum rotational torque that can be expressed by the transmission member 4 can be substantially higher than the first torque and in detail substantially between the first torque and the sum of said first torque and said second torque.

Preferably in the high-thrust configuration, the maximum rotational speed that can be expressed by the transmission member 4 is substantially lower than the first and in detail almost no higher than the second speed.

In summary, the kinematic mechanism 43 is configured to selectively define or release a rotational constraint between the actuators 41 and 42. In low-thrust configuration, the kinematic motion 43 does not define the rotational constraint between actuators 41 and 42, whereas in high-thrust configuration it defines the rotational constraint between the actuators 41 and 42.

Preferably, said kinematic mechanism 43 is configured to selectively define or release a rotational constraint in only one direction of rotation.

The kinematic mechanism 43 may comprise a first wheel **431** configured to receive output motion from the first actuator 41; a second wheel **432** configured to receive output motion from the second actuator 42; and a connector **433** configured to selectively allow or prevent a passage of motion between the wheels 431 and 432. The first wheel 431 can be attached to a coupling and in particular to the first attachment 2.

Preferably the wheels 431 and 432 are pulleys.

The second wheel can have a substantially larger radius than the first wheel.

The connector 433 is configured to allow the second wheel 432 (i.e., the second actuator 42) to control rotation between the attachments 2 and 3, and to be precise between the prosthetic joint 1 and the prosthesis 1a exclusively via the first wheel 431 (i.e., the first actuator 41).

In a low-thrust configuration, the connector 433 prevents a passage of motion between the wheels 431 and 432 preferably for at least said angle of rotation. It may thus allow the first wheel 431 to rotate (suitably by at least said angle of rotation) while leaving the second wheel 432 substantially stationary.

In a high thrust configuration, the connector 433 allows a passage of motion between the wheels 431 and 432 preferably for at least said angle of rotation. It may thus force the wheels 431 and 432 to rotate synchronously by angles proportional to each other in accordance with the rays and/or suitably for at least said angle of rotation.

In a preferred form of non-limiting embodiment, the connector 433 can be identified in at least one cable subtended between the wheels 431 and 432, and appropriately each wheel 431 and 432 can be a pulley to which said cable is tied at the throat.

In order to have a passage of motion between the wheels 431 and 432 (i.e. between the actuators 41 and 42) in only one direction of rotation, the connector 433 can be identified as a single cable with one end integral with the first wheel 431, the other end integral with the second wheel 432 and a single subtended portion between the two wheels 431 and 432.

The cable can be made of synthetic fibres such as high-modulus polyethylene (HMPE), also known as ultra-high molecular weight polyethylene (UHMWPE, UHMW) commercially known under the brand name Dyneema^{®}.

The length of the connector 433 can be substantially equal to the distance between the rotation axes of the wheels 431 and 432, to the portion of cable wound on each wheel; and to the arc defined by the radius of a wheel 431 and 432 (in detail the second one) multiplied by the rotation angle of said wheel corresponding to the rotation angle of the prosthetic joint 1.

In a low-thrust configuration, the cable (i.e. connector 433) can be slack so that the first wheel 431 can rotate relative to the second wheel 432 by exploiting this condition of the cable. In a high thrust configuration, the cable may be taut so that a rotation between the prosthesis 1a and 1b is only possible by producing a synchronous movement of the wheels 432 and 431.

In order to hold the cable (i.e. the connector 433) in the grooves, the kinematic mechanism 43 may comprise a tensioner **434** configured to hold the connector 433 in place (in detail in the grooves).

The tensioner 434 is configured to provide the cable (i.e. the connector 433) with a minimum voltage substantially lower than the standstill voltage of the second actuator 42, i.e. lower than the resistance of the second actuator 42 to change from a standstill condition to a movement condition.

The tensioner 434 may comprise an arm **434a** integral with the coupling 3, a thrust wheel **434b** on the connector 433; an additional arm **434c** hinging the thrust wheel 434b cantilevered from the arm 434ab; and a spring configured to work in opposition to a cable tension. Said spring being preloaded so as to provide said minimum tension to the cable.

The functioning of the active prosthetic joint 1 described above in structural terms is as follows.

Initially, the prosthetic joint 1 is in a low thrust configuration and therefore the kinematic mechanism 43 does not define the rotational constraint between actuators 41 and 42. The cable (i.e. connector 433) is slack leaving the second actuator 42 kinematically disconnected from both actuators 41 and 42 the only first actuator 41 kinematically connected to an attachment 2.

In this configuration, the prosthetic joint 1 can express a thrust torque and velocity that are substantially proportional to the first torque and velocity, respectively. When the user must, for example, confront a climb, he commands the joint 1 to switch to a high-thrust configuration.

Therefore, the second actuator 42 rotates the second wheel 432 which wraps around the connector 433 tensioning it. Appropriately, during this time the first actuator 41 and thus the first wheel 431 can remain substantially stationary.

This operation is performed in opposition to the tensioner 434 as the connector 433, by pressing on the thrust wheel 434b, rotates the additional arm 434c in relation to the arm 434a in opposition to the spring.

At this point, the kinematic mechanism 43 defines the rotational constraint between actuators 41 and 42. The cable (i.e. connector 433) is tensioned by kinematically connecting both connections 2 and 3.

In detail, the actuators 41 and 42 are constrained by the kinematic mechanism 43 to rotate in unison in one direction of rotation for at least said angle of rotation. Thus, the actuators 41 and 42 can discharge their torque on the joint 1, which is then able to express a maximum rotational torque given by the sum of the first and second torque.

It should also be noted that since both actuators 41 and 42 are connected to each other, the maximum achievable speed is limited to that of the second actuator 42.

Once the high-thrust configuration is reached, the user can tackle the ascent.

Once the ascent is complete, there is a return to a low-thrust configuration.

The second actuator 42 rotates the second wheel 42 in the opposite direction to the previous one by unwinding the connector 433, which becomes slack. Appropriately at this time the first actuator 41 and thus the first wheel 431 can remain substantially stationary.

At the same time, the tensioner 434, utilising the energy previously stored, tensions the connector 433) to said minimum voltage, i.e. to a voltage lower than that at which the second actuator 42 can remain stationary while the first actuator 41 moves.

The invention introduces an innovative movement procedure implementable by means of the active prosthetic joint 1 according to the invention.

The movement process may include at least one movement phase in which the prosthetic joint 1 is in a low thrust configuration or in a high thrust configuration.

In each movement phase the movement member 4, using the first actuator 41 and in some cases the second actuator 42, moves the joint 1 and thus a rotation between the prostheses 1a and 1b and in detail between the prosthetic joint 1 and the prosthesis 1a.

Preferably, it comprises several movement phases that differ from each other in the configuration assumed by the prosthetic joint 1. For example, the procedure may sequentially comprise a first movement phase with joint 1 in a low thrust configuration, a second handling phase with joint 1 in a high-thrust configuration, and a third movement phase with the joint 1 in a low thrust configuration.

In the first and third thrust phase, only the first actuator 41 is kinematically connected to attachment 2 and thus only it controls the rotation between prostheses 1a and 1b and thus between prosthetic joint 1 and prosthesis 1a.

In the second thrust phase, both actuators 41 and 42 are kinematically connected to at least one attachment 2 and/or 3, and thus both the first actuator 41 and the second actuator 42 control said rotation.

The movement process may include at least one configuration change phase in which the prosthetic joint 1 changes its configuration between low thrust and high thrust

In each configuration change step, the kinematic mechanism 43 selectively defines or releases the aforementioned rotational constraint between actuators 41 and 42 by controlling a reciprocal motion between the actuators 41 and 42.

This is performed by means of a rotation between the actuators 41 re 42 and thus between the wheels 431 and 432, which respectively make the connector 433 tight or slack.

Preferably, the process comprises a configuration change phase interposed between two adjacent movement phases. Thus, with reference to the aforementioned example, the procedure may sequentially comprise a first handling phase with the joint 1 in a low thrust configuration; a first configuration change phase in which the joint 1 switches from a low thrust configuration to a high thrust configuration; a second movement phase in which the prosthetic joint 1 is in a high thrust configuration; a second configuration change phase in which the joint 1 switches from a high thrust configuration to a low thrust configuration; and a third handling phase in which the joint 1 is in a low thrust configuration.

In the first phase of the configuration change, the second actuator 42 commands a rotation of the second wheel 432, which wraps the connector 433 by tensioning it and thus realising said rotational constraint between the actuators 41 and 42. In this first phase, the first actuator 41 and thus the first wheel 431 can remain stationary. In the second configuration change phase, the second actuator 42 commands a rotation of the second wheel 432 in the opposite direction to that of the first configuration change phase. Said rotation of the second wheel 432 unwinds the connector 433 by keeping it and thereby releasing said rotational constraint. In this second phase, the first actuator 41 and thus the first wheel 431 can remain stationary.

The active prosthetic joint 1 and thus the movement process according to the invention achieve important advantages.

In fact, the prosthetic joint 1 and thus the handling procedure are able to vary their configuration between low thrust and high thrust in a simple and practical way, ensuring optimal power according to the power required for the specific activity. One advantage is that the prosthetic joint 1 and thus the movement procedure are simple and convenient to operate and use.

Another important advantage is the fact that, compared to known active prosthetic joints, the prosthetic joint 1 is smaller in size and in detail almost comparable to a normal joint.

A not insignificant advantage is to be found in the reduced weight of the prosthetic joint 1.

A further advantage is the mechanical simplicity of the prosthetic joint 1, which therefore has low costs in both production and maintenance.

## Claims

1. Prosthetic joint (1) configured to mutually rotate a first prosthesis (1a) and a second prosthesis (1b) and comprising:
- an attachment (2) of said prosthetic joint (1) to said first prosthesis 1a);
- an additional attachment (3) of said prosthetic joint (1) to said second prosthesis (1b);
- a movement member (4) configured to control a rotation between said attachments (2, 3) and therefore between said prostheses (1a, 1b) defining a thrust torque for said rotation;
and wherein said movement member (4) comprises
- a first actuator (41) defining a first pair for said rotation;
- a second actuator (42) defining for said rotation a second torque substantially greater than said first torque; and
- a kinematic mechanism (43) configured to exploit said actuators (41, 42) to control said rotation; **characterised in that**
said kinematic mechanism (43) is configured to selectively define or dissolve a rotational constraint between said actuators (41, 42) by defining
o a low thrust configuration in which said first actuator (41) is kinematically connected to said attachment (2) and said second actuator (42) is kinematically disconnected from said attachment (2) so that exclusively said first actuator (41) controls said rotation defining a maximum rotation torque substantially not greater than said first torque and
o a high thrust configuration in which both said actuators (41, 42) are kinematically connected to said attachment (2) so that both said first actuator (41) and said second actuator (42) control said rotation defining a maximum rotation torque substantially comprised between said first pair and the sum of said first pair and said second pair.

2. Prosthetic joint (1) according to claim 1, in which in said high-thrust configuration said actuators (41, 42) are kinematically connected in one direction of rotation only.

3. Prosthetic joint (1) according to at least one preceding claim, wherein said first torque is substantially between 75 Nm and 25 Nm and said second torque is substantially between 150 Nm and 50 Nm.

4. Prosthetic joint (1) according to at least one preceding claim, wherein said first actuator (41) defining a first speed for said rotation and said second actuator (42) defining for said rotation a second speed substantially lower than said first speed; and wherein in said low thrust configuration said rotation defines a maximum rotation speed substantially not higher than said first speed and in said high thrust configuration said rotation defines a maximum rotation speed substantially not higher than said second speed.

5. Prosthetic joint (1) according to the preceding claim, wherein said first speed is substantially comprised between 100 rpm and 50 rpm and said second speed is substantially comprised between 50 rpm and 15 rpm.

6. Prosthetic joint (1) according to at least one preceding claim, wherein said kinematic mechanism (43) comprises a first wheel (431) configured to receive the output motion from said first actuator (41), a second wheel 432 configured to receive the motion in output from said second actuator (42) and a connector 433 configured to prevent a passage of motion between said wheels (431; 432) in said low thrust configuration and to allow a passage of motion between said wheels (431; 432) in said high thrust configuration.

7. Prosthetic joint (1) according to the preceding claim, in which said connector (433) is a single cable having a first end integral with said first wheel (431), a second end integral with said second wheel (432) and a single underlying portion between the two wheels (431, 432).

8. Prosthetic joint (1) according to the previous claim, in which said cable is made of synthetic fibres such as high modulus polyethylene.

9. Prosthetic joint (1) according to at least one claim 7-8, wherein said cable is slack in said low thrust configuration and stretched in said high thrust configuration.

10. Movement process comprising a prosthetic joint (1) according to at least one preceding claim; said movement process comprising
- a plurality of differentiated movement phases due to the configuration assumed by said prosthetic joint (1);
- at least one configuration change step, each of which is interposed between two of said movement phases; in each of said at least one configuration change phase said kinematic mechanism (43) controlling a rotation between said actuators (41, 42)
o defines said rotational constraint between said actuators (41, 42) by placing both said first actuator (41) and said second actuator (42) kinematically connected to said attachment (2) so that both said actuators (41, 42) control said rotation defining a maximum rotation torque substantially comprised between said first pair and the sum of said first pair and said second pair, or
o dissolves said rotational constraint between said actuators (41, 42) by placing said first actuator (41) kinematically connected to said attachment (2) and said second actuator (42) is kinematically disconnected from said attachment (2) so that exclusively said first actuator (41) controls said rotation defining a maximum rotation torque substantially not higher than said first torque

## Patentansprüche

1. Prothetisches Gelenk (1), das dazu konfiguriert ist, eine erste Prothese (1a) und eine zweite Prothese (1b) gegenseitig zu drehen, und umfassend:
- eine Befestigung (2) des prothetischen Gelenks (1) an der ersten Prothese (1a);
- eine zusätzliche Befestigung (3) des prothetischen Gelenks (1) an der zweiten Prothese (1b);
- ein Bewegungselement (4), das dazu konfiguriert ist, eine Drehung zwischen den Befestigungen (2, 3) und somit zwischen den Prothesen (1a, 1b) zu steuern und dabei ein Schub-Drehmoment für die Drehung zu definieren;
und wobei das Bewegungselement (4) umfasst:
- einen ersten Aktuator (41), der ein erstes Drehmoment für die Drehung definiert;
- einen zweiten Aktuator (42), der ein zweites Drehmoment für die Drehung definiert, das wesentlich größer als das erste Drehmoment ist; und
- einen kinematischen Mechanismus (43), der dazu konfiguriert ist, die Aktuatoren (41, 42) zum Steuern der Drehung zu nutzten, **dadurch gekennzeichnet, dass**
der kinematische Mechanismus (43) dazu konfiguriert ist, selektiv eine Drehungsbegrenzung zwischen den Aktuatoren (41, 42) zu definieren oder zu beseitigen, indem er
∘ eine Konfiguration mit niedrigem Schub, in der der erste Aktuator (41) kinematisch mit der Befestigung (2) verbunden ist und der zweite Aktuator (42) kinematisch von der Befestigung (2) getrennt ist, so dass der erste Aktuator (41) ausschließlich die Drehung steuert, wobei ein maximales Drehmoment definiert wird, das im Wesentlichen nicht größer als das erste Drehmoment ist;
∘ eine Konfiguration mit hohem Schub, in der beide Aktuatoren (41, 42) kinematisch mit der Befestigung (2) verbunden sind, so dass sowohl der erste Aktuator (41) als auch der zweite Aktuator (42) die Drehung steuern, wobei ein maximales Drehmoment definiert wird, das im Wesentlichen zwischen dem ersten Drehmoment und der Summe des ersten Drehmoments und des zweiten Drehmoments liegt, definiert.

2. Prothetisches Gelenk (1) nach Anspruch 1, wobei die Aktuatoren (41, 42) in der Konfiguration mit hohem Schub nur in einer Drehrichtung kinematisch verbunden sind.

3. Prothetisches Gelenk (1) nach mindestens einem der vorhergehenden Ansprüche, wobei das erste Drehmoment im Wesentlichen zwischen 75 Nm und 25 Nm liegt und das zweite Drehmoment im Wesentlichen zwischen 150 Nm und 50 Nm liegt.

4. Prothetisches Gelenk (1) nach mindestens einem der vorhergehenden Ansprüche, wobei der erste Aktuator (41) eine erste Geschwindigkeit für die Drehung definiert und der zweite Aktuator (42) für die Drehung eine zweite Geschwindigkeit definiert, die im Wesentlichen niedriger als die erste Geschwindigkeit ist; und wobei die Drehung in der Konfiguration mit niedrigem Schub eine maximale Drehgeschwindigkeit definiert, die im Wesentlichen nicht höher als die erste Geschwindigkeit ist, und wobei die Drehung in der Konfiguration mit hohem Schub eine maximale Drehgeschwindigkeit definiert, die im Wesentlichen nicht höher als die zweite Geschwindigkeit ist.

5. Prothetisches Gelenk (1) nach dem vorhergehenden Anspruch, wobei die erste Geschwindigkeit im Wesentlichen zwischen 100 U/min und 50 U/min liegt und die zweite Geschwindigkeit im Wesentlichen zwischen 50 U/min und 15 U/min liegt.

6. Prothetisches Gelenk (1) nach mindestens einem vorhergehenden Anspruch, wobei der kinematische Mechanismus (43) ein erstes Rad (431) umfasst, das dazu konfiguriert ist, die Ausgangsbewegung von dem ersten Aktuator (41) zu empfangen, ein zweites Rad (432), das dazu konfiguriert ist, die Ausgangsbewegung von dem zweiten Aktuator (42) zu empfangen, und ein Verbinder (433), der dazu konfiguriert ist, in der Konfiguration mit niedrigem Schub eine Bewegungsübertragung zwischen den Rädern (431; 432) zu verhindern und in der Konfiguration mit hohem Schub eine Bewegungsübertragung zwischen den Rädern (431; 432) zu ermöglichen.

7. Prothetisches Gelenk (1) nach dem vorhergehenden Anspruch, wobei der Verbinder (433) ein einziges Kabel ist, das ein erstes Ende aufweist, das einstückig mit dem ersten Rad (431) ausgebildet ist, ein zweites Ende, das einstückig mit dem zweiten Rad (432) ausgebildet ist, und einen einzigen darunterliegenden Abschnitt zwischen den zwei Rädern (431, 432).

8. Prothetisches Gelenk (1) nach dem vorhergehenden Anspruch, wobei das Kabel aus synthetischen Fasern wie hochmoduligem Polyethylen besteht.

9. Prothetisches Gelenk (1) nach mindestens einem der Ansprüche 7-8, wobei das Kabel in der Konfiguration mit niedrigem Schub lose ist und in der Konfiguration mit hohem Schub gespannt wird.

10. Bewegungsverfahren umfassend ein prothetisches Gelenk (1) nach mindestens einem vorhergehenden Anspruch; das Bewegungsverfahren umfassend:
- eine Vielzahl differenzierter Bewegungsphasen aufgrund der von dem prothetischen Gelenk (1) angenommenen Konfiguration;
- mindestens einen Konfigurationswechselschritt, wobei jeder zwischen zwei der Bewegungsphasen angeordnet ist; wobei in jeder der mindestens einen Konfigurationswechselschritte der kinematische Mechanismus (43) eine Drehung zwischen den Aktuatoren (41, 42) steuert, indem er
o die Rotationsbegrenzung zwischen den Aktuatoren (41, 42) definiert, indem er sowohl den ersten Aktuator (41) als auch den zweiten Aktuator (42) kinematisch mit der Befestigung (2) verbindet, so dass beide Aktuatoren (41, 42) die Drehung steuern und ein maximales Drehmoment definieren, das im Wesentlichen zwischen dem ersten Drehmoment und der Summe des ersten Drehmoments und des zweiten Drehmoments liegt, oder
o die Rotationsbegrenzung zwischen den Aktuatoren (41, 42) beseitigt, indem er den ersten Aktuator (41) kinematisch mit der Befestigung (2) verbindet und der zweite Aktuator (42) kinematisch von der Befestigung (2) trennt, so dass der erste Aktuator (41) ausschließlich die Drehung steuert und ein maximales Drehmoment definiert, das im Wesentlichen nicht höher als das erste Drehmoment ist.

## Revendications

1. Articulation prothétique (1) configurée pour faire tourner mutuellement une première prothèse (1a) et une seconde prothèse (1b), et comprenant :
- un élément de fixation (2) de ladite articulation prothétique (1) à ladite première prothèse (1a) ;
- un élément de fixation supplémentaire (3) de ladite articulation prothétique (1) à ladite seconde prothèse (1b) ;
- un élément de mouvement (4) configuré pour commander une rotation entre lesdits éléments de fixation (2, 3) et donc entre lesdites prothèses (1a, 1b), définissant un couple de poussée pour ladite rotation ;
et où ledit élément de mouvement (4) comprend :
- un premier actionneur (41) définissant un premier couple pour ladite rotation ;
- un second actionneur (42) définissant pour ladite rotation un second couple sensiblement supérieur audit premier couple ; et
- un mécanisme cinématique (43) configuré pour exploiter lesdits actionneurs (41, 42) pour commander ladite rotation,
**caractérisé en ce que**
ledit mécanisme cinématique (43) est configuré pour définir ou annuler sélectivement une contrainte de rotation entre lesdits actionneurs (41, 42) en définissant :
∘ une configuration à faible poussée dans laquelle ledit premier actionneur (41) est relié cinématiquement audit élément de fixation (2) et ledit second actionneur (42) est déconnecté cinématiquement dudit élément de fixation (2), de sorte que seul ledit premier actionneur (41) commande ladite rotation en définissant un couple de rotation maximal sensiblement non supérieur audit premier couple ; et;
∘ une configuration à forte poussée dans laquelle lesdits actionneurs (41, 42) sont reliés cinématiquement audit élément de fixation (2), de sorte que tant ledit premier actionneur (41) que ledit second actionneur (42) commandent ladite rotation en définissant un couple de rotation maximal sensiblement compris entre ledit premier couple et la somme dudit premier couple et dudit second couple.

2. Articulation prothétique (1) selon la revendication 1, dans laquelle, dans ladite configuration à forte poussée, lesdits actionneurs (41, 42) sont reliés cinématiquement dans une seule direction de rotation.

3. Articulation prothétique (1) selon au moins une des revendications précédentes, dans laquelle ledit premier couple est sensiblement compris entre 75 Nm et 25 Nm, et ledit second couple est sensiblement compris entre 150 Nm et 50 Nm.

4. Articulation prothétique (1) selon au moins une des revendications précédentes, dans laquelle ledit premier actionneur (41) définit une première vitesse pour ladite rotation et ledit second actionneur (42) définit pour ladite rotation une seconde vitesse sensiblement inférieure à ladite première vitesse ; et dans laquelle, dans ladite configuration à faible poussée, ladite rotation définit une vitesse de rotation maximale sensiblement non supérieure à ladite première vitesse, et dans ladite configuration à forte poussée, ladite rotation définit une vitesse de rotation maximale sensiblement non supérieure à ladite seconde vitesse.

5. Articulation prothétique (1) selon la revendication précédente, dans laquelle ladite première vitesse est sensiblement comprise entre 100 tr/min et 50 tr/min, et ladite seconde vitesse est sensiblement comprise entre 50 tr/min et 15 tr/min.

6. Articulation prothétique (1) selon au moins une des revendications précédentes, dans laquelle ledit mécanisme cinématique (43) comprend une première roue (431) configurée pour recevoir le mouvement de sortie dudit premier actionneur (41), une seconde roue (432) configurée pour recevoir le mouvement de sortie dudit second actionneur (42) et un connecteur (433) configuré pour empêcher un passage de mouvement entre lesdites roues (431 ; 432) dans ladite configuration à faible poussée et pour permettre un passage de mouvement entre lesdites roues (431 ; 432) dans ladite configuration à forte poussée.

7. Articulation prothétique (1) selon la revendication précédente, dans laquelle ledit connecteur (433) est un câble unique présentant une première extrémité solidaire de ladite première roue (431), une seconde extrémité solidaire de ladite seconde roue (432) et une portion unique sous-jacente entre les deux roues (431, 432).

8. Articulation prothétique (1) selon la revendication précédente, dans laquelle ledit câble est constitué de fibres synthétiques telles que du polyéthylène à module élevé.

9. Articulation prothétique (1) selon au moins une des revendications 7-8, dans laquelle ledit câble est détendu dans ladite configuration à faible poussée et tendu dans ladite configuration à forte poussée.

10. Procédé de mouvement comprenant une articulation prothétique (1) selon au moins une des revendications précédentes ; ledit procédé de mouvement comprenant :
- une pluralité de phases de mouvement différenciées en raison de la configuration assumée par ladite articulation prothétique (1) ;
- au moins une étape de changement de configuration, chacune étant interposée entre deux desdites phases de mouvement ; dans chacune desdites au moins une phase de changement de configuration, ledit mécanisme cinématique (43) commandant une rotation entre lesdits actionneurs (41, 42)
o définit ladite contrainte de rotation entre lesdits actionneurs (41, 42) en connectant cinématiquement tant ledit premier actionneur (41) que ledit second actionneur (42) audit élément de fixation (2), de sorte que les deux actionneurs (41, 42) commandent ladite rotation en définissant un couple de rotation maximal sensiblement compris entre ledit premier couple et la somme dudit premier couple et dudit second couple ; ou
o annule ladite contrainte de rotation entre lesdits actionneurs (41, 42) en connectant cinématiquement ledit premier actionneur (41) audit élément de fixation (2) et en déconnectant cinématiquement ledit second actionneur (42) dudit élément de fixation (2), de sorte que seul ledit premier actionneur (41) commande ladite rotation en définissant un couple de rotation maximal sensiblement non supérieur audit premier couple.
